# EUROPEAN PATENT SPECIFICATION

(11) **EP 2 558 148 B1**
(45) Date of publication and mention of the grant of the patent: **13.04.2016**
(21) Application number: 11720618.5
(22) Date of filing: 13.04.2011
(51) Int. Cl.: A61M 15/00

(54) **DRY POWDER INHALER MOUTHPIECE BUTTON**
TROCKENPULVERINHALATOR-MUNDSTÜCK
BOUTON D'EMBOUT BUCCAL POUR INHALATEUR À POUDRE SÈCHE

(30) Priority: 26.04.2010 TR 201003238; 20.04.2010 TR 201003091; 13.04.2010 TR 201002877
(43) Date of publication of application: 20.02.2013
(73) Proprietor: Sima Patent ve Lisanslama Hizmetleri Ltd.Sti., Esenler/Istanbul (TR)
(72) Inventor: BILGIC, Mahmut, Merter/Istanbul 34173 (TR)
(86) International application number: PCT/TR2011/000093
(87) International publication number: WO 2011/129793

(56) References cited:
- WO-A1-02/36189
- WO-A1-2009/003989
- GB-A- 2 447 560
- US-A1- 2009 078 252

## Description

### Field of the Invention

The present invention relates to an inhalation device used in delivering medicament indry powder form to patients by the inhalation route. In addition, the present invention relates to an inhalation device providing delivery of dry powder medicament as stored in blister packages effectively and safely, and the method for using said device.

### Description of the Prior Art

It is well known to use inhalation devices for delivering medicaments utilized in the treatment and prophylaxis of respiratory diseases by the inhalation route. Inhalation treatment is the most commonly preferred treatment method in these diseases as the inhalation devices provide ease of use; the medicaments have a more rapid onset of time resulting from local administration and they have fewer side effects. Inhalation devices have been designed in order to provide effective and sufficient delivery of the medicaments used in the treatment of respiratory diseases, particularly in asthma and chronic obstructive pulmonary disease. These inhalation devices vary according to their operating mechanisms and the physical form of the medicament to be delivered.

In the inhalation devices used to deliver drugs in dry powder form, the drug is carried in reservoirs, capsules or blisters packages. It is important to deliver each dose to the patient with exact accuracy and preciseness since the required medicament dose in the inhalation is very low. Inhalation devices comprising blister packages, which are one of the inhalation device types utilized for the inhalation of the medicaments in dry powder form mentioned above, are commonly used. The blister package is comprised of a plurality of blister pockets each of which contains medicament in dry powder form. In response to each actuation of the inhalation device to realize the inhalation, the blister package is indexed from one part of the device to the other; one of the blister pockets is opened and one dose of the dry powder medicament becomes ready for inhalation subsequent to the delamination of the blister package or the perforation of it by piercing components in the device.

It is highly significant to deliver a sufficient amount of the dry powder medicament contained in the blister pocket which is opened to realize an effective inhalation in the inhalation devices comprising blister packages to the lungs in each actuation. In addition to the dry powder medicament's physical and chemical characteristics such as suitable aerodynamic particle size, suitable particle shape, uniformity of the particle size distribution, low aerodynamic dispersion forces, low density, high physical and chemical stability, the mechanism and/or the specifications of the inhalation device are of the factors affecting the efficiency of the inhalation. The strong effect of the medicaments, which are used in inhalation treatment, at low doses highlights the significance of controlled dosing in each inhalation during the inhalation treatment.

Underdosing of the dry powder medicament administered to the patient during inhalation operation results in the absence of desired effects while overdosing results in undesirable side effects. Thus, it is required that the necessary amount of the dry powder medicament is delivered to the patient during inhalation for the desired effect to be observed in the treatment.

Due to the fact that the medicaments in dry powder form which are utilized in inhalation treatment have strong effects even on very low amounts and have serious side effects, the accidental use of these medicaments by healthy people, particularly by children, might be dangerous. In addition, inadvertent actuation of the device out of inhalation time may cause the blisters to be vainly opened and the dry powder medicament in the blisters to spill into the device, which results in uncontrolled dose intake.

A wide range of inhalation devices have been designed to enable inhalation of the medicament in dry powder form from blisters.

Some of the devices present in the state of the art are summarized below;

WO 2009/003989 discloses an inhaler suitable for delivery of the medicament in dry powder form from a blister package composed of a plurality of blister pockets. Said device comprises a mouthpiece, a gear mechanism, a rotatable mouthpiece cover, a drive gear, a housing and a pawl to prevent movement of the mouthpiece cover.

US 2009/078252 discloses a fault sensing system for dry powder inhalers. The document mainly aims to detect breakage or some other sort of failure (such as stretching or delamination or tearing) of the blister strip to prevent the user from using the device although it no longer delivers the dry powder.

GB 2447560 discloses a dry powder inhaler wherein the device is actuated upon movement of the mouthpiece such that it is easy to use and the dose is not exposed to moisture and contamination from the air as te seal is broken only prior to use.

WO02/36189 discloses a medicament dispenser comprising; a body, a holder shaped to fit within said body and moveable relative to the body, a cassette containing medicament carrierwherein movement of the holder relative to the body results in movement of the cassette between a first position and a second position such that the cassette is reversibly removable from the holder when the cassette is in the second position.

According to this, the inventor has designed aninhalation device which is suitable for delivery of the medicament in dry powder form from a blister package and enables controlled dose intake of the medicament in dry powder form during each inhalation.

The present invention relates to an inhalation device which guarantees to eliminate the problems mentioned above. Therefore, the present invention relates to a safe inhalation device which has been designed so as not to enable inadvertent and accidental actuation and provide to deliver the dry powder medicament to the patient with controlled dosing.

The present invention also relates to an inhalation device which is suitable for delivery of the medicament in dry powder form from a blister package and has a mechanism that guarantees the blister package to be accurately and precisely positioned in response to each actuation of the device.

The present invention also relates to an inhalation device having a mechanism which provides the patient to make sure that the required amount of the dry powder medicament for the treatment is inhaled during each inhalation.

### Summary of the Invention

According to the present invention, an inhalation device suitable for delivery of the medicament in dry powder form from a blister package composed of a plurality of blister pockets which are spaced at equal intervals, each of which comprises medicament in dry powder form; the inhalation device comprising:
a mouthpiece enabling the patient to inhale the medicament in dry powder form from the opened blister in use;
a gear mechanism enabling in use the blister package to be indexed and the medicament in dry powder form to become ready for inhalation;
a rotatable mouthpiece cover hiding the mouthpiece and triggering the gear mechanism;
a drive gear which is a component of the gear mechanism and is joined with the mouthpiece cover via a connection point of a mouthpiece cover, and
a housing situated between an upper housing member and a lower housing member in which the blister package and the gear mechanism are enclosed, and
a stopper for preventing the movement of the mouthpiece cover characterized in that:
   the stopper consists of a pressing button, a pawl and a supporting part; wherein the pawl is engaged with the recess part on the inside surface of the mouthpiece cover to prevent the movement of the mouthpiece cover when the mouthpiece cover is in a position in which the mouthpiece is completely covered, and the pressing button that moves synchronously with the pawl is pressed to enable the mouthpiece cover to be rotated for the actuation of the inhalation device as the supporting part is sprung to allow the pressing button to be pressed.

The housing of the device of the present invention has been designed such that each component of the blister package and the gear mechanism, which have a significant role in enabling the device to work properly, is situated accurately and works harmoniously. To this end, the housing is divided into several compartments. The used portion and the unused portion of the blister package are accommodated in separated compartments in order to prevent the medicament in dry powder form remaining in the opened blister pocket spilling the other components of the housing. Furthermore, the housing also comprise a beak which enables the blister package to be peeled and a manifold through which the dry powder medicament in the open blister passes before reaching the mouthpiece during inhalation. The housing can be in any appropriate shape, while it is preferably elliptic or circular.

An upper and a lower housing members interlock with each other and enclose the housing in order to keep the housing and the gear mechanism fixed together. The mouthpiece cover hiding the mouthpiece is rotated by being slid on the upper and lower housing members. Carved parts on the surface of the lower and the upper housing members provide effective actuation by preventing the slipping of the finger while rotating the mouthpiece cover. The upper and the lower housing members can be in any appropriate shape which provides ease of use.

The mouthpiece cover hiding the mouthpiece of the device of the present invention has been designed such that it also actuates the device. When the upper and the lower housing members of the device are joined together, engagement tabs on the inside surface of the lower housing member engage with engagement recesses on the inside surface of the upper housing member and the upper and lower housing members are fixed tightly. In addition, the protrusions on the upper and the lower housing members are joined end to end and form a restricted path where the mouthpiece cover rotates. Before each inhalation, both the mouthpiece is uncovered and one dose of the medicament in dry powder form becomes ready for inhalation as one of the blister pockets is opened as a result of the mouthpiece cover being manually rotated along the path restricted by joining the protrusions on the upper and the lower housing members. The rotational path on which the cover moves is restricted on both ends by the protrusions of the upper and the lower housing members. The constant-distance path that the protrusions of the upper and the lower housing members define results in the mouthpiece cover being rotated by a fixed angle in the range of 30° to 160°, preferably, 50° to 120°, most preferably 55°, 60°, 65°, 70°, 75°, 80° to 92.5°, 95°, 97.5°, 100°, 102.5°, 105°, 107.5°, 110°, 112.5°, 115° in response to each actuation of the device.

The mouthpiece cover that triggers the gear mechanism of the device can be found in one of two positions. After pressing the pressing button of the stopper, the mouthpiece cover can easily be switched from the first position to the second position. When the mouthpiece cover is in the first position, the mouthpiece cover resides on the protruding part in one end of the rotational path. When the first position is on, the mouthpiece cover is completely covered and the device is in standby mode. When the mouthpiece cover is in the second position, the mouthpiece cover resides on the protruding part in the other end of the rotational path and one dose of the dry powder medicament becomes ready for inhalation upon the actuation of the device.

In each actuation of the inhalation device, if the blister package is not indexed or positioned properly less than the required amount of the dry powder medicament is inhaled as the blister pocket is not opened completely; or more than the required amount of the dry powder medicament is inhaled if more than one blister pocket is opened which may result in dangerous complications for the patient. Therefore, it is highly significant to provide the blister to be positioned at an accurate position and to be completely opened in each actuation of the inhalation device.

According to the present invention, there are protective covers on the connection points of the mouthpiece cover. Each of the protective covers comprises a fixing part and an extension part. These protective covers are fixed to the inhalation device by the fixing parts that are present under the protective covers and engage with holes on the upper and lower housing member of the inhalation device. The extension parts that are present at the centre of the inside surface of the protective covers enable the mouthpiece cover to connected with the inhalation device by passing through the connection points of the mouthpiece cover.

The mouthpiece cover has two connection points. One of them is on one side of the mouthpiece cover and the other is on another side of the mouthpiece cover to enable the mouthpiece cover to be connected with the inhalation device. The mouthpiece cover of the inhalation device is joined with the gear mechanism by the drive gear via one connection point of the mouthpiece cover. Only one end of the drive gear is connected with one connection point of the mouthpiece cover while the other end of the drive gear is not connected to the mouthpiece cover at any points. The end of the drive gear that is fixed into one connection point of the mouthpiece cover has a hole at the centre. In addition, each of the connection points itself is a hole in shape. Therefore, the end of the drive gear is passing through the connection point of the mouthpiece cover while the extension part present at the centre of the inside surface of the protective cover is passing through both the centre of said end of the drive gear and the connection point of the mouthpiece cover. Shape of the one connection point of the mouthpiece cover is different from shape of other connection point of the mouthpiece cover as well as shape of the end of the drive gear that is connected to one connection point of the mouthpiece cover is different from shape of other end of the drive gear.

The shape of the end of the drive gear that matches with the connection point of the mouthpiece cover through which said end of the drive gear passes, has surface area in the range of 30-100 mm² preferably in the range of 32-90 mm² and more preferably in the range of 40-80 mm² and particularly in the range of 45, 46, 47, 48, 49, 50, 51, 52, 53, 54, 55 to 60, 61, 62, 63, 64, 65, 66, 67, 68, 69, 70, 71, 72, 73, 74, 75 mm². Adjustment of the surface area to the range given above enables transmission of the force of at least 5 Newton that is applied to afford the rotational movement of the mouthpiece cover to be transmitted to the gear mechanism accurately. In addition, the shape of the end of the drive gear is preferably quadrangular, most preferably a trapezoid, with an surface area in defined range. Therefore, the one end of the drive gear with the surface area in the range of 30-100 mm² passes through one connection point of the mouthpiece cover and this enables the rotational movement of the mouthpiece cover to be transmitted to the gear mechanism accurately via only one end of the drive gear and enables the mouthpiece cover synchronize with the drive gear.

Each of the protective cover present on one of the connection point of the mouthpiece cover is fixed to the inhalation device by the fixing part. A guiding tooth on the inside surface of each of the protective covers is guided in the guiding path that is above the connection point while the mouthpiece cover is rotated for the actuation of the inhalation device over the restricted rotational path. The guiding teeth are passed through the holes on the upper housing member and the lower housing member as well as they are on the inside surface of the protective covers.

The other connection point of the mouthpiece cover that is not connected to any end of the drive gear is related to the inhalation device via the extension part of the protective cover that passes through said connection point of the mouthpiece cover as well as centre of the upper housing member. In addition, the connection point of the mouthpiece cover that is connected to the end of the drive gear with the surface area in the range of the 30-100 mm² is related to the inhalation device via the other extension part of the protective cover that passes through said connection point of the mouthpiece cover as well as centre of the lower housing member.

In each actuation of the inhalation device, the gear mechanism is triggered by the rotation movement of said mouthpiece cover from the first position to the second position along the rotational path restricted on both ends by the protrusion parts on the upper and the lower housing members to provide the blister package to be advanced. In each actuation of the device, the constant-distance path that the protrusions of the upper and the lower housing members define allows said mouthpiece cover to be rotated by a fixed angle. Accordingly, because of the fact that the one end of the drive gear that matches with the connection point of the mouthpiece cover through which said end passes is in a shape that has a surface area in the range of 30-100 mm², in each actuation of the inhalation device, only rotation of said mouthpiece cover from the first position to the second position on the constant-distance path guarantees that the blister package is advanced by the same distance and the opened blister is at accurate position so that the sufficient amount of the dry powder formulation contained in the opened blister can be inhaled. In addition to this, the patient is sure about whether the blister is opened completely or not by controlling the position of the mouthpiece cover on the constant-distance path. In other words, if the mouthpiece cover is in the second position wherein it resides on the protrusion part on the other end of the constant-distance rotational path, the patient is sure about that one blister is completely opened and one dose of the dry powder medicament contained in the opened blister becomes ready for inhalation; if the mouthpiece cover is between the first position and the second position, the patient is sure about that one blister is not completely opened.

According to the present invention, each gear of the gear mechanism in the device directly or indirectly engages with each other. The drive gear, which is one of the components of the gear mechanism, provides the mouthpiece cover to trigger the gear mechanism. In each actuation of the device, the constant-angle rotational movement of the cover is transmitted to gear of an indexing ratchet wheel which interlocks with a indexing wheel via the drive gear. The indexing wheel synchronizes with the indexing ratchet wheel when the mouthpiece cover is switched from the first position to the second position. The gear of the indexing wheel is engaged with a winding wheel gear and a pinion gear and the rotation of the indexing wheel gear causes them to move as well. A mechanism wheel engages with the inside of the winding wheel gear via the arms of the mechanism wheel. A counter gear that engages with a small gear under a base gear rotates by means of the base gear that engages with a pinion gear and a counter gear with movement of the mouthpiece cover. Therefore, with the rotation of the indexing wheel, both the lid sheet of the blister package is provided to be coiled on the wings of the winding wheel as the winding wheel is rotated by the mechanism wheel and the counter gear rotates to display the new number of unused blister from the display aperture.

According to the present invention, the indexing wheel may be another component of the gear mechanism. The recesses of the indexing wheel match with the shape of the blister package. The fact that the blister pockets of the blister package are received in these recesses in sequence while the indexing wheel rotates allows the blister package to be indexed properly. The rotation angle of the indexing wheel depends on the number of the recesses of the indexing wheel. In each actuation of the inhalation device, the indexing wheel can be rotated by the angle of 15° to 120°. According to the invention, there are preferably 8 recesses of the indexing wheel. Therefore, the indexing wheel is supposed to rotate by the angle of 45° in response to each actuation of the device for the opened blister pocket to be positioned accurately. The drive gear which is connected with the mouthpiece cover accurately transmits the constant rotational movement, by an angle in the range of 30° to 160°, preferably, 50° to 120°, most preferably 55°, 60°, 65°, 70°, 75°, 80° to 92.5°, 95°, 97.5°, 100°, 102.5°, 105°, 107.5°, 110°, 112.5°, 115°, of the mouthpiece cover to the gear of the indexing ratchet wheel when the device is actuated. As the arms of the indexing ratchet wheel interlock with the indexing wheel from the inside, the indexing wheel and the indexing ratchet wheel synchronize. Therefore, the constant-angle rotation that the mouthpiece cover executes when the device is actuated causes the indexing wheel to rotate by a constant angle of 45°. The length of the path that the mouthpiece cover follows and the constant angle value is adjusted such that the indexing wheel rotates by 45° in response to each actuation of the device. Thus, the blister package is completely opened for the inhalation of the sufficient amounts of the medicament in dry powder form as a result of the accurate positioning of the blister package in response to each actuation of the device of the present invention.

The inhalation device of the present invention has a stopper that is situated between the lower housing member and the housing as engaging with the two holes on the lower housing member. This stopper consists of a pressing button, a pawl and a supporting part. Each of the pressing button and the pawl passes through one hole of the lower housing member and is shown from the outside as the supporting part is situated at the inside of the inhalation device and is not shown from the outside. According to the present invention, for actuation of the inhalation device, the mouthpiece cover is switched from the first position to the second position over the rotational path. However, the pawl is engaged to the recess part on inside surface of the mouthpiece cover to prevent the movement of the mouthpiece cover when the mouthpiece cover is in the first position in which the mouthpiece is completely covered. Since the pressing button moves synchronously with the pawl, when the pressing button is pressed, the pawl is advanced forward inside of the inhalation device and disengaged from the mouthpiece cover. After the pawl is disengaged from the mouthpiece cover, the mouthpiece cover can be rotated from the first position to the second position to actuate the inhalation device.

Because of the supporting part of the stopper, there is no need for a spring to enable the pressing button to be pressed. There is an end that is integrated with the supporting part and leans the housing of the inhalation device. When the pressing button is pressed, the supporting part springs over the end of said supporting part and both of the pressing button and the pawl are advanced forward inside of the inhalation device.

Additionally, at least one component which is situated in the lower housing member and serves as another stopper engages with the teeth of at least one of the gears of the gear mechanism and stabilizes the gear in a suitable position in order to prevent backward rotation of the blister package. While the stopper can hinder the rotation of any gear of the gear mechanism, it preferably hinders the rotation of the indexing wheel connected with the indexing ratchet wheel. The stopper can be anywhere suitable in the lower housing member and have any shape. Consequently, the inhalation device according to the present invention has two stopper that provide the blister package to be accurately and precisely positioned in response to each actuation of the device as preventing the accidental actuation of the inhalation device. Thus, controlled dosing of the dry powder medicament in the opened blister pocket is enabled when the device is actuated.

The term "controlled dosing" refers to the intake of the medicament in dry powder form on the required amount.

The counter gear in the device pertaining to the present invention displays the number of the unused blister pockets remaining in the device. In response to the actuation of the device by the mouthpiece cover, the mouthpiece is uncovered, the blister package is indexed and one dose of the dry powder medicament is prepared for inhalation while the counter gear rotates as well.

On the counter gear, there exist numerals equal to the number of the blister pockets present in the device and they are spaced at equal angles. In a device comprising 60 doses, the angle between the numerals is approximately 5°. The counter gear rotates as a result of the transmission of the rotation of the indexing wheel gear via the pinion gear and the base gear. In response to the each actuation of the device, rotation of the indexing wheel by the same angle each time due to the accurate transmission of the movement of the mouthpiece cover to the gear mechanism via the drive gear results in the rotation of the counter gear approximately by the same angle as well and each numeral on the counter gear is clearly seen through the display aperture on the upper housing member. Therefore, the patient is sure of the number of the unused blister pockets remaining in the device.

Before the inhalation, the device is actuated by the mouthpiece cover being rotated along the rotational path, and the drive gear with the surface area in the range of 30-100 mm² of one end which is joined with the one connection point of the mouthpiece cover precisely transmits the movement of the mouthpiece cover to the gear of the indexing ratchet wheel. The indexing ratchet wheel interlocks with the indexing wheel from inside via its arms and enables the indexing wheel to rotate 45° in each actuation of the device. Upon the indexing wheel's movement, the blister package is indexed and is peeled by means of the beak present in the housing. As the indexing wheel gear engages with the winding wheel gear and the pinion gear, these gears synchronize with the indexing wheel. Since the mechanism wheel engages both with the winding wheel gear and the winding wheel, the rotation of the winding wheel gear causes the winding wheel to move and the lid sheet of the blister package coils tightly on the winding wheel. The pinion gear engages with the base gear while the small gear under the base gear engages with the counter gear. In brief, the movement of the mouthpiece cover along the rotational path causes the blister package to be accurately positioned as a result of the rotation of the indexing wheel by 45° and the number of the unused blisters remaining in the device is seen through the display aperture as the counter gear rotates by 5°.

The inhalation device according to the present invention comprises a blister package composed of a plurality of blister pockets each of which comprises medicament in dry powder form and which are spaced at equal intervals. The blister package carries the medicament in dry powder form in one-dose portions and it is preferably a blister strip and it is preferably peelable. The blister pockets comprised in the blister package are spaced in equal intervals and each of them carries one dose of the medicament in dry powder form.

While the blister package is indexed on the indexing wheel, the beak on the housing peels the blister. Therefore, one dose dry powder medicament becomes ready for inhalation after the blister package is peeled open in each actuation of the device.

The base sheet of the blister package on which the blister cavities are spaced is accumulated in the separated compartment of the housing. The lid sheet that provides seal of the blister package, on the other hand, is coiled on the winding wheel, which is one of the components of the gear mechanism positioned in the other side of the housing. The winding wheel has a plurality of resilient wings. The resilient wings of the winding wheel has been designed such that they balance the force of attraction on the lid sheet of the blister package applied by the winding wheel by eliminating the augmentation emerging in the diameter of the winding wheel as a result of the lid sheet of the blister package coiling on the wings of the winding wheel. The rotation of the winding wheel is induced by the rotation of the winding wheel gear, which the winding wheel engages with, by the indexing wheel when the device is actuated. The mechanism wheel that engages with the winding wheel gear causes the winding wheel to rotate unidirectionally.

The blister pocket that is opened as a result of the beak's peeling the blister package is situated immediately under the manifold. Upon the inhalation by the patient, the airflow that enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that enables external air to enter the device easily and at convenient speed.

The air inlet that the external air flow passes through is preferably designed not to be close where the patient holds the device in order not to prevent air flow. Furthermore, in order to deliver the required amount of the dry powder medicament in the opened blister to the patient, the air inlet is designed such that it allows the entry of the airflow through the air inlet at a convenient angle.

One end of the manifold communicates with the opened blister while the other end communicates with the inlet. On the one end of the manifold that communicates with the blister, at least two apertures with four sub-apertures are situated. Upon the inhalation of the patient, some of the air flow which enters through the air inlet passes through one of these apertures and entrains the medicament in dry powder form through the other aperture to the manifold. There is preferably a tapered channel between the manifold and the mouthpiece to connect these components each other. The medicament in dry powder form that is entrained to the manifold with the airflow is delivered to the patient via the mouthpiece. The manifold can be in any appropriate shape though it is preferably longer than 1mm.

The mouthpiece is designed to fit the mouth for the patient to comfortably inhale the medicament in dry powder form. According to the shape of the device, the mouthpiece can be in any suitable shape or size as well as being fixed or movable. Furthermore, it could be attached or unattached to the upper and/or the lower cover.

Each component of the device pertaining to the present invention can be made of any suitable material though they are preferably be made of plastic. These plastics can preferably be chosen from a group comprising the types of styrene acrylonitrile, polyoxymethylene acetale, acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer while it is preferably polyoxymethylene acetale. The plastic components can be manufactured by methods like injection molding. Moreover, each component can be in any suitable color.

According to the present invention, the lid and base sheets composing the blister package are sealed very tightly by at least one of the methods comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive. According to the present invention, the lid and the base sheets constituting the blister package preferably consist of a plurality of layers. Polymeric layers, aluminum foil and preferably fluoropoylmer film are among the layers that form the lid and the base sheet. Fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

In order to provide high moisture and gas protection, aluminum foil can be used in lid and base sheets of blister packs. These aluminum foils must be thick enough to provide the desired protection for the stability of the moisture sensitive dry powder formulation stored in the blister cavity while they can be chosen to be preferably in the range of 5 to 80 µm, more preferably in the range of 15 to 65 µm.

The polymeric layers in the lid and the base sheets of the blister pack mentioned in the present invention are made of the same or different polymers. The thickness of these polymeric layers varies according to the type of the polymeric substance used and its properties while they are preferably in the range of 5-100 µm, more preferably in the range of 15-60 µm.

In the blister package, the layer which is in contact with the dry powder medicament in the blister cavity is preferably a polymeric layer. Due to the porous structure of aluminum foil and electrostatic forces, some of the dry powder formulation sticks onto the inside layer of the blister cavity, and hence it may cause uncontrolled dosing.

The term "uncontrolled dosing" refers to the intake of the medicament in dry powder form less or more than the required amount.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be the same or different shape, structure or volume.

The reference numbers of the drawings added to exemplify the present invention and the detailed description of the invention according to these drawings are given below but the scope of the invention should not be limited to these drawings.

### In the Drawings

Figure 1 is a perspective view of an inhalation device according to the present invention;
Figure 2 is a view of the mouthpiece cover exploded from the inhalation device;
Figure 3 is a perspective view of the blister pack for use with the inhalation device of the invention;
Figure 4 is a perspective view of the housing and the gear mechanism of the inhalation device according to the invention;
Figure 5 is another perspective view of the housing and the gear mechanism of the inhalation device of the invention;
Figure 6 is a perspective view of the housing of the inhalation device of the invention;
Figure 7 is a perspective view of the upper housing member of the inhalation device of the invention;
Figure 8 is a perspective view of the lower housing member of the inhalation device of the invention;
Figure 9 is a perspective view of the mouthpiece cover of the inhalation device of the invention;
Figures 10a and 10b are perspective views of the drive gear of the inhalation device of the invention;
Figure 11 is a perspectiveview of the connection of in the mouthpiece cover, the drive gear and the protective covers of the inhalation device of the invention;
Figure 12 is a perspective of the connection between mouthpiece cover and the protective covers of the inhalation device of the present invention;
Figure 13 is a perspective view of the connection between the mouthpiece cover, the drive gear, the lower housing member and the protective cover of the inhalation device of the present invention;
Figures 14a and 14b are perspective views of the stopper consisting of the pawl, the pressing button and the supporting member used in the inhalation device of the present invention;
Figure 15 is a perspective view of the connection between the indexing wheel and another stopper used in the inhalation device of the invention.

### Detailed Description of the Drawings

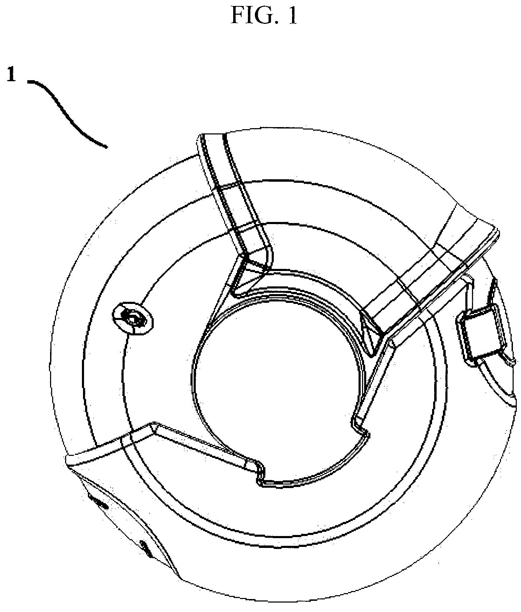

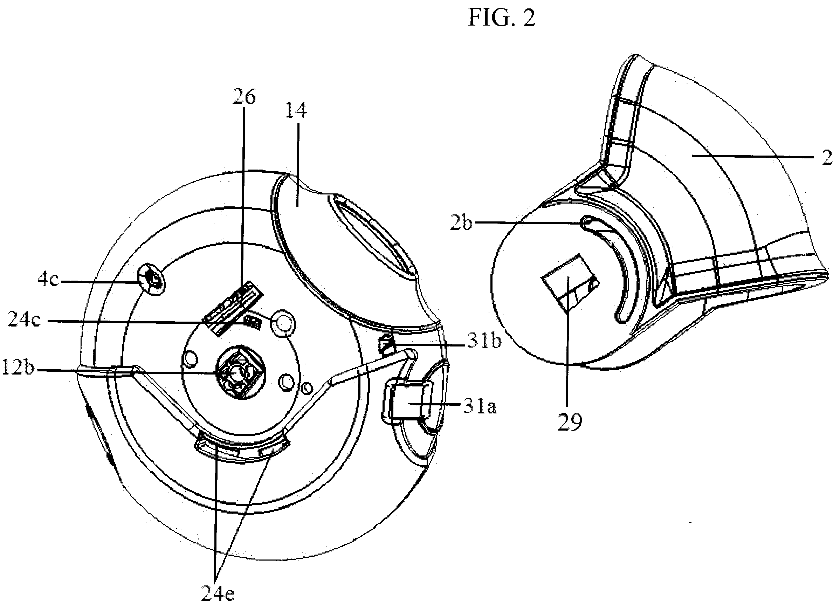

The inhalation device (1) of the present invention shown in figure 1 and 2 comprises a gear mechanism situated in the housing (10) between an upper housing member (4a) and a lower housing member (4b) in order to enable the inhalation of the dry powder medicament carried in a blister package (15). Each component of the inhalation device (1) is positioned at particular spots in the housing (10) to guarantee proper and accurate working.

The inhalation device (1) of the present invention shown in figure 1 is ready for actuation. In this case, a mouthpiece cover (2) is in a first position and the mouthpiece (2) is completely covered. The mouthpiece cover (2) is rotated by holding one end of the mouthpiece cover (2) in order to switch to the first position, wherein the mouthpiece is completely covered, from the second position. In this way, the mouthpiece (14) is completely exposed while the mouthpiece cover (2) is switched from the first position to the second position and the gear mechanism is triggered by a drive gear (12). The drive gear (12) precisely transmits the movement of the mouthpiece cover (2) to an indexing ratchet wheel (3).

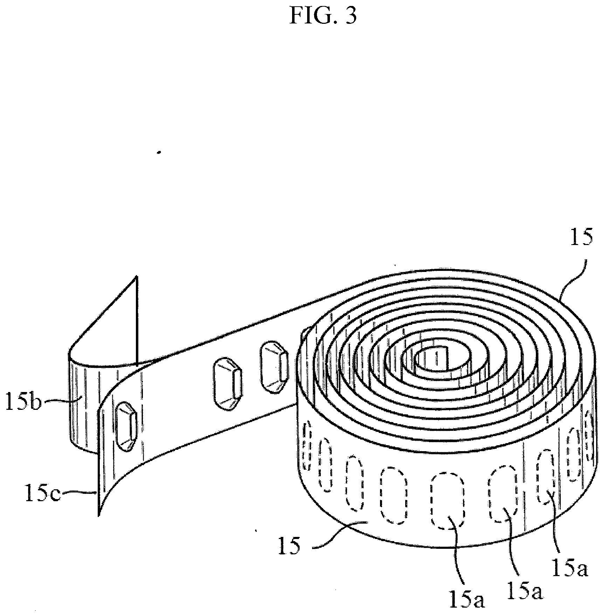

An indexing wheel (8) engages with the indexing ratchet wheel (3) and enables the blister package (15) shown in figure 3 to be indexed. Blister pockets (15a) composing the blister package are received in recesses (8a) on the indexing wheel and the blister package (15) is indexed when the indexing wheel (8) rotates. In the inhalation device (1) of the present invention, the shapes of these recesses (8a) on the indexing wheel (8) have been designed to match with the shapes of the blister pockets (15) composing the blister package (15) for the blister package to be indexed properly.

The blister package (15) shown in figure 3 is composed of a lid sheet (15b) which provides sealing and a base sheet (15c) on which the blister pockets (15a) are spaced at equal intervals. Each blister pocket contains medicament in dry powder form comprising one or more active agents.

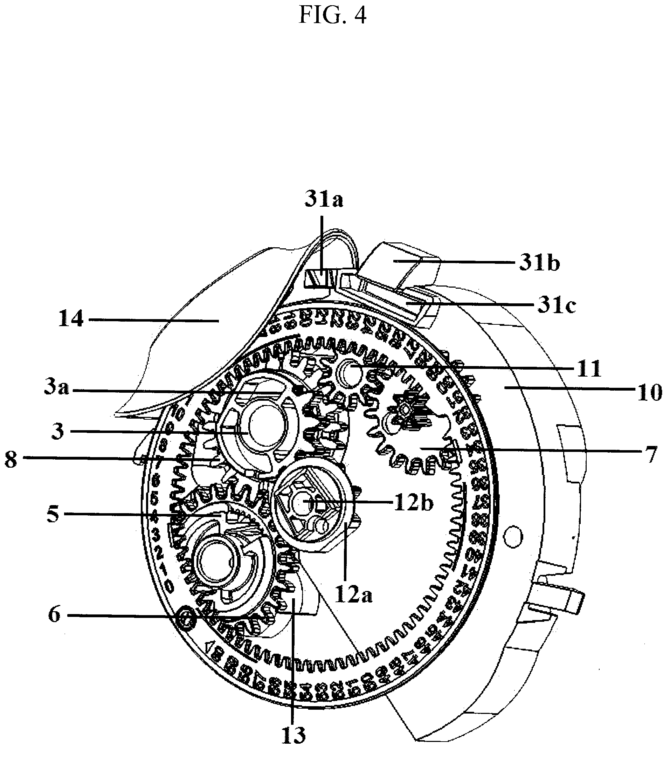

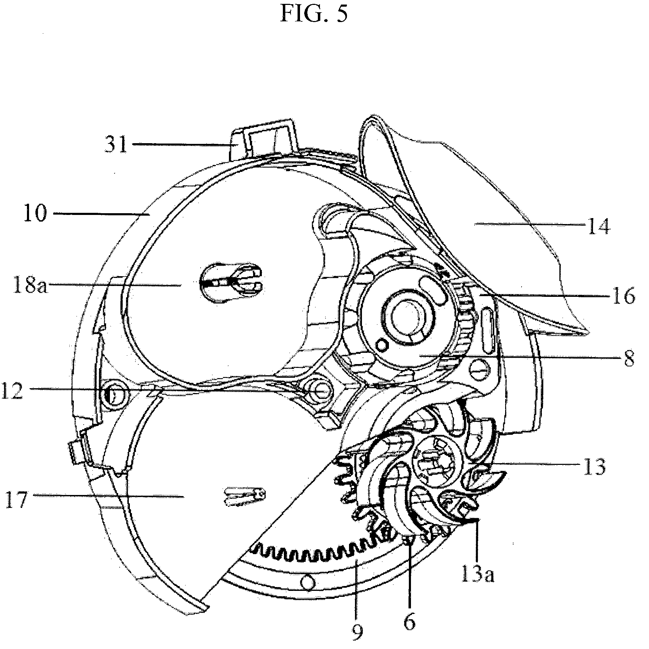

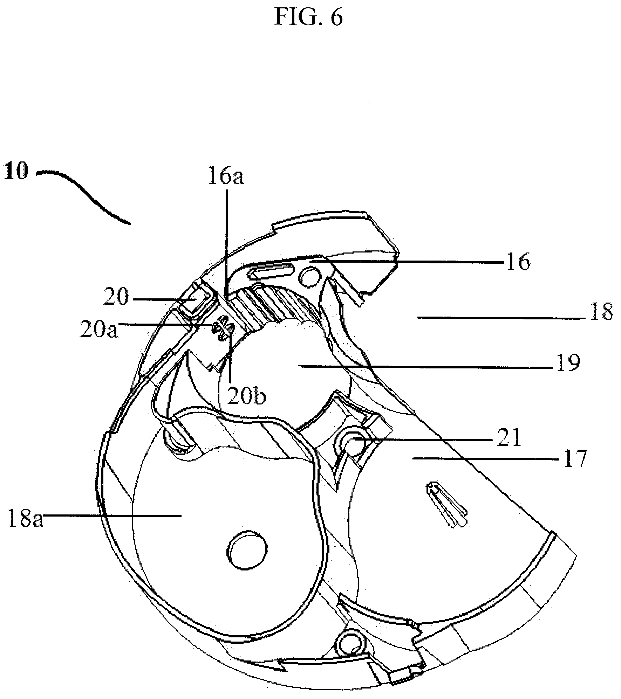

The rotational movement that the mouthpiece cover (2) of the device for actuation of the inhalation device executes while switching from the first position to the second is transmitted to the indexing ratchet wheel (3) via the drive gear (12) that the mouthpiece cover (2) engages with. As displayed in figure 4, arms (3a) of the indexing ratchet wheel interlock with the indexing wheel (8) from inside and rotate the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. A beak (16) shown in the figure 5 in the housing (10) peels the blister package (15) while the blister package (15) is indexed and provides one blister pocket (15a) to be opened in response to each actuation of the device (1). The beak (16) peels the blister package (15) with the help of a sharp edge (16a) that is in contact with the lid sheet of the blister package (15b) (figure 6).

A winding wheel gear (6), which is another component of the gear mechanism, engages with the gear of the indexing wheel (8) as displayed in figure 4. A mechanism wheel (5) that interlocks the winding wheel (13) from inside has arms (5a) to interlock with the interior teeth of the winding wheel gear (6). Therefore, the mechanism wheel (5) engages with both the winding wheel (13) and the winding wheel gear (6) and moves with these component in actuation of the inhalation device (1). When the indexing wheel (8) rotates the winding wheel gear (6), the winding wheel (13) rotates unidirectionally owing to the arms (5a) of the mechanism wheel interlocking with the interior teeth of the winding wheel gear (6) and the lid sheet (15b), which is peeled away by the beak (16) while the blister package is indexed, is tightly coiled on the wings (13a) of the winding wheel. The base sheet (15c) of the blister package (15) where the blister pockets are spaced is accumulated in a separate part (18a) of the device (figure 5).

As can be seen in figures 5 and 6, the housing (10) also comprises components having significant roles in the actuation of the device such as the beak (16), the manifold (20), and the apertures with four sub-apertures (20a, 20b). Each component comprised in the housing is situated in an appropriate part of the housing (10) in order to enable the inhalation device (1) to work properly. The drive gear (12) passes through the center (21) of the housing and engages with the mouthpiece cover (2) at one connection point. The blister package (15) is in the lower part (17) of the housing and coiled up. In response to each actuation of the device (1), the blister package (15) is peeled by the beak (16) in the housing while being indexed by the indexing wheel (8) situated in the upper part (19) of the housing. The lid sheet (15b) of the blister package (15), which provides sealing, is indexed over the beak (16) and coiled on the wings of the winding wheel (13a) which is situated in the side part (18) of the housing. The base sheet (15c) of the blister package (15) on which the blister pockets (15a) are spaced, on the other hand, is accumulated in the separated compartment (18a) of the housing (10). Upon the inhalation by the patient, air passes through the aperture with four sub-apertures (20a), under the manifold (20) and into the opened blister pocket. The air entrains the dry powder medicament contained in the opened blister pocket (15a) in response to each actuation of the device; and passes through the other aperture with four-sub-apertures (20b) and reach the mouthpiece via the manifold (20).

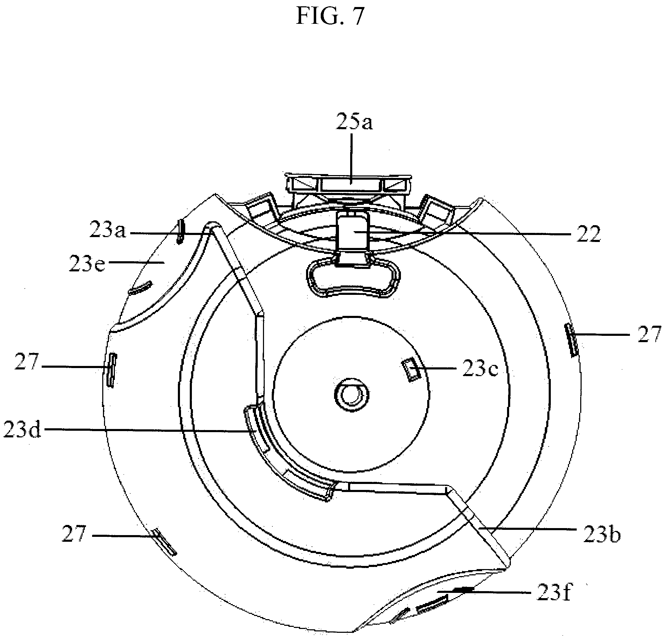

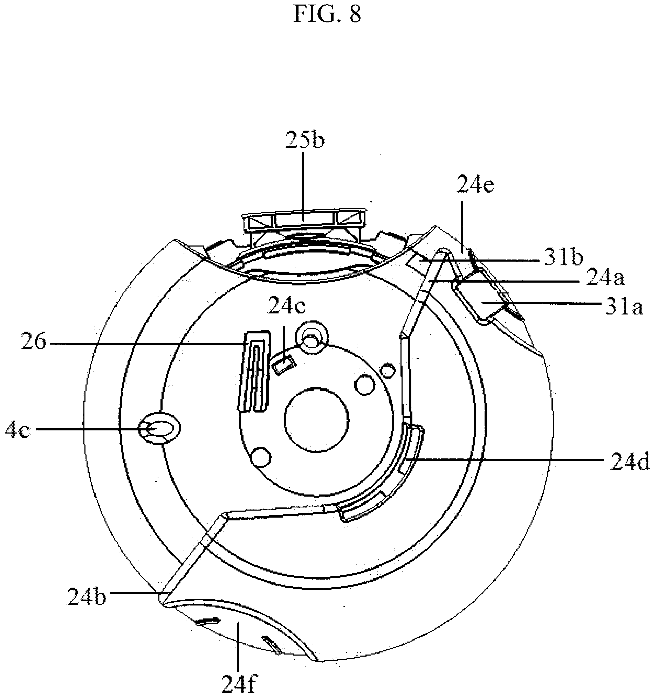

The housing (10) and the other components of the inhalation device (1) pertaining to the present invention are kept together as the upper housing member (4a) and the lower housing member (4b) displayed in figures 7 and 8 are joined together. Engagement tabs (28) on the inside surface of the lower housing member (4b) engage with engagement recesses (27) on the inside surface of the upper housing member (4a) and the upper and lower housing members are fixed tightly. Therefore, the protrusions (23a; 23b) on the upper housing member (4a) and the protrusions (24a, 24b) on the lower housing member (4b) are joined end to end and define a restricted path for the rotational movement of the mouthpiece cover (2). In each actuation of the device, the constant-distance path that the protrusions (23a, 23b, 24a, 24b) of the upper and the lower housing members (4a, 4b) define allows said mouthpiece cover (2) to be rotated by a fixed angle. The mouthpiece cover (2) can be moved from the first position to the second position along this restricted path. When the mouthpiece cover (2) is in the first position, the mouthpiece (14) is completely covered, the device is in standby mode and the mouthpiece cover (2) leans on a first protrusion (23a) on the upper housing member and the first protrusion (24a) on the lower housing member (figure 1). The mouthpiece (14) is manually slid along the rotational path to switch to the second position. The mouthpiece (14) is completely exposed when the cover (2) is in this position, one dose of the dry powder medicament is ready for inhalation and the mouthpiece cover (2) leans on a second protrusion (23b) on the upper housing member and a second protrusion (24b) on the lower housing member.

As displayed in figures 7 and 8, one half (25a) of the tapered channel that interconnects the manifold (20) with the mouthpiece (14) is comprised in the upper housing member (4a) while the other half of it (25b) is comprised in the lower housing member (4b). The channel is constituted as a whole when the upper (4a) and the lower (4b) housing members are joined together. Upon the inhalation by the patient, air that enters the device through at least one air inlet (22) arranged in the upper housing member (4a) passes through the aperture with four sub-apertures (20a), reaches the opened blister (15a) and entrains the dry powder medicament there to the manifold (20) by passing it through the other aperture with four sub-apertures (20b). Carved parts on the upper housing member (23e, 23f) and carved parts on the lower housing member (24e, 24f) prevent a slip of the finger while rotating the mouthpiece cover.

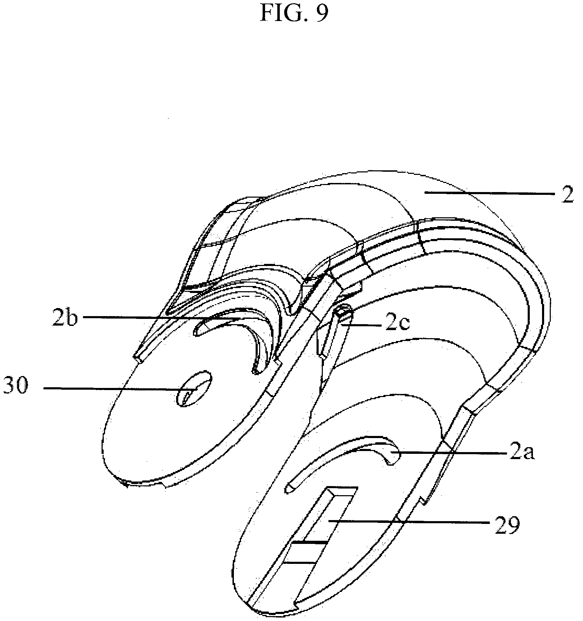

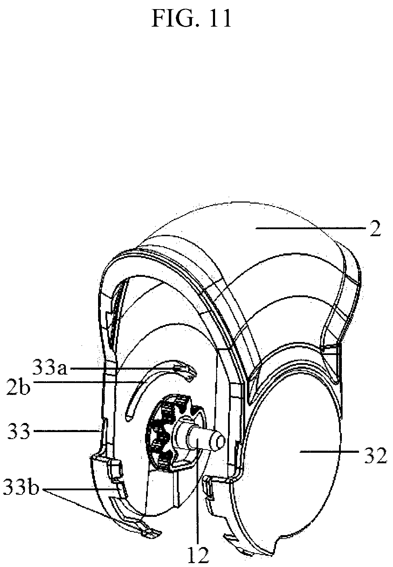

The mouthpiece cover (2) of the inhalation device pertaining to the present invention is displayed in figure 9. The mouthpiece cover (2) is joined with the gear mechanism via connection points (29, 30). The drive gear (12) is joined with one connection points (29) of the mouthpiece cover as can be seen in figure 11, illustrating the connection between the mouthpiece cover (2) and the drive gear (12)

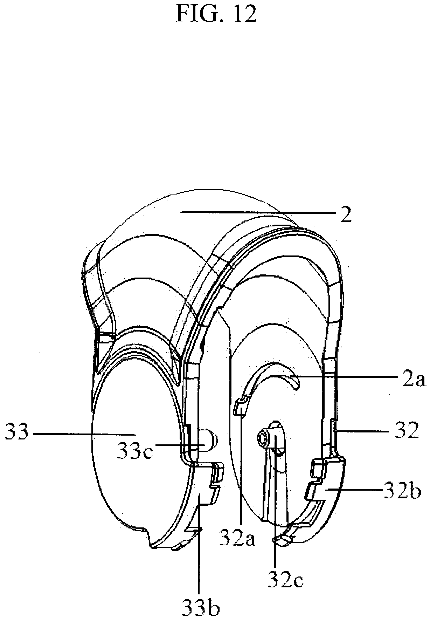

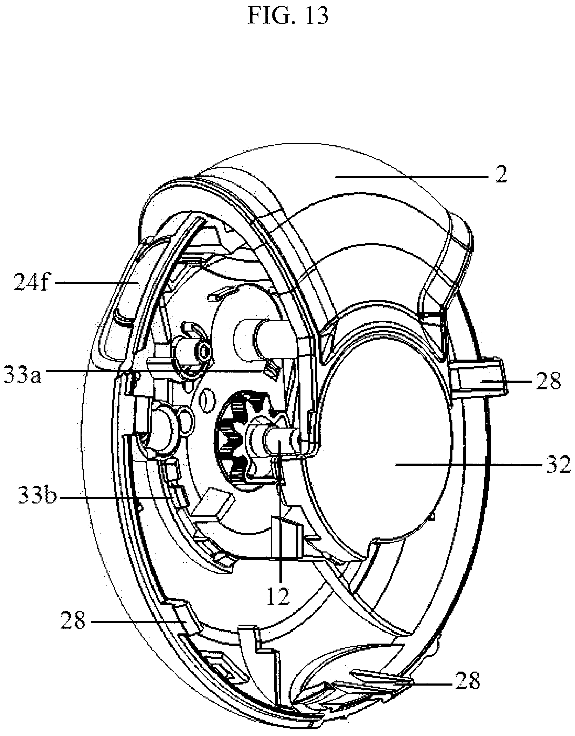

There are protective covers (32, 33) on the connection points of the mouthpiece cover (29, 30) as can be seen figures 11, 12 and 13. Each of the protective covers comprises a fixing part (32b, 33b) and an extension part (32c, 33c). These protective covers (32, 33) are fixed to the inhalation device (1) by the fixing parts (32b, 33b) that are present under the protective covers (32, 33) and engage with the holes (23d, 24d) on the upper and lower housing member of the inhalation device (figures 7 and 8). The extension parts (32c, 33c) that are present at the centre of the inside surface of the protective covers (32, 33) enable the mouthpiece cover (2) to be connected with the inhalation device by engaging with the connection points (29, 30) of the mouthpiece cover.

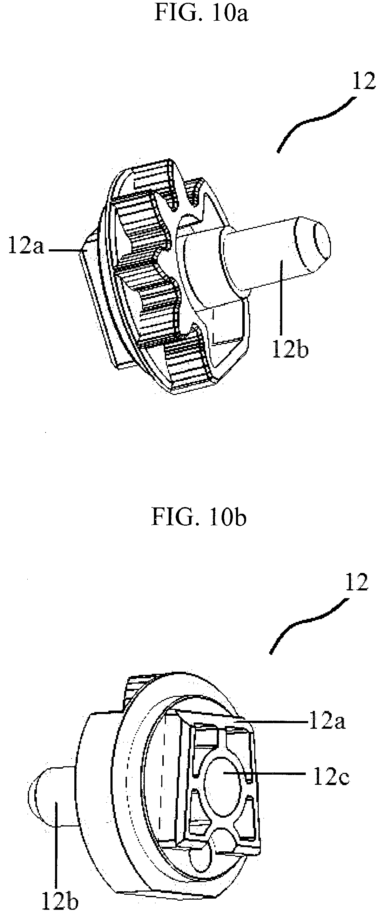

The mouthpiece cover (2) has two connection points, one of them (29) is on one side of the mouthpiece cover and other (30) is on another side of the mouthpiece cover, to enable the mouthpiece cover to be connected to the inhalation device (figure 9). The drive gear (12) consists of two ends (12a, 12b) and a hole (12c) at the centre of one end (figure 10a and 10b). The mouthpiece cover of the inhalation device is joined with the gear mechanism by the drive gear (12) via one connection point (29) of the mouthpiece cover (2) (figure 11). Only one end of the drive gear (12a) is connected to one connection point of the mouthpiece cover (29) while the other end of the drive gear (12b) is not connected to the mouthpiece cover (2) at any points (figure 11). The end of the drive gear (12a) that is fixed into one connection point of the mouthpiece cover (29) has a hole (12c) at the centre (Figure 10b). In addition, each of the connection points (29, 30) itself is a hole in shape Figure 9). Therefore, the end of the drive gear (12a) is fixed into the connection point of the mouthpiece cover (29) while the extension part (33c) present at the centre of the inside surface of the protective cover is fixed into both the hole (12c) at the centre of said end of the drive gear and the connection point of the mouthpiece cover.

The shape of one of the connection points of the mouthpiece cover (29) is different from the shape of the other connection point of the mouthpiece cover (30) as well as the shape of the end of the drive gear (12a) that is connected with one connection point of the mouthpiece cover (29) is different from shape of other end of the drive gear (12b) (figures 9, 10a and 10b).

Each of the protective covers (32, 33) present on one of the connection points of the mouthpiece cover (29, 30) is fixed to the inhalation device by the fixing part (32b, 33b). A guiding tooth (32a, 33a) on the inside surface of each of the protective covers (32, 33) is guided on the guiding path (2a, 2b) that is above the connection point (29, 30) while the mouthpiece cover (2) is rotated for the actuation of the inhalation device over the restricted rotational path. The guiding teeth (32a, 33a) is passed through the holes (23c, 24c) on the upper housing member (4a) and the lower housing member (4b) as well as they are on the inside surface of the protective covers (32, 33).

The mouthpiece cover (2) rotates by the same angle each time on the constant-distance path that is composed by splicing the protrusions (23a, 23b) on the upper housing member and the protrusions (24a, 24b) on the lower housing member illustrated in figures 7 and 8 while switching from the first position to the second position. Since oneend of the drive gear (12a) with the defined surface area interlocks with the connection point (29) of the mouthpiece cover the mouthpiece cover (2) and the drive gear (12) synchronize and the rotational movement of the mouthpiece cover (2) by a constant angle in response to each actuation of the device causes the drive gear (12) to rotate by a constant angle each time. Thus, the rotation of the drive gear (12) by a constant angle causes the indexing wheel (8) to rotate by the same angle via the indexing ratchet wheel (3) in response to each actuation of the device.

On the indexing wheel (8) illustrated in figures 4 and 5, there are preferably 8 recesses (8a) and the angles between these recesses are equal. Therefore, the indexing wheel (8) rotates by 45° each time the device is actuated for the blister pockets that are received in these recesses (8a) to be positioned accurately. The blister package (15) indexed by the 45° rotation of the indexing wheel (8) in response to each actuation of the device is peeled by the beak (16) and one dose of the dry powder medicament becomes ready for inhalation when one blister pocket is opened.

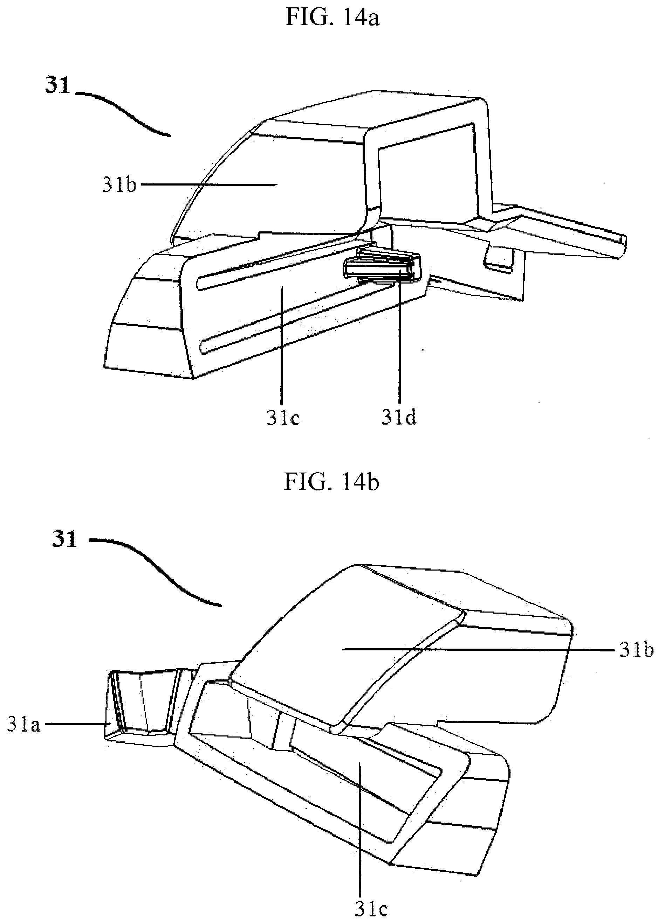

The inhalation device (1) of the present invention has a stopper (31) that is situated between the lower housing member (4b) and the housing (10) as engaging with the two holes on the lower housing member (4b) (figures 2 and 8). Said stopper is shown in figures 14a and 14b. This stopper (31) consists of a pawl (31a), a pressing button (31b), and a supporting part (31c). Each of the pawl (31a) and the pressing button (31b) situated in one hole of the lower housing member (4b) and is shown from the outside as the supporting part (31c) is situated at the inside of the inhalation device and is not shown from the outside (figures 2 and 4). For actuation of the inhalation device, the mouthpiece cover (2) is switched from the first position to the second position over the rotational path. However, the pawl (32a) is engaged to the recess part (2c) on inside surface of the mouthpiece cover (2) to prevent the movement of the mouthpiece cover (2) when the mouthpiece cover (2) is in the first position in which the mouthpiece (14) is completely covered. Since the pressing button (31b) moves synchronously with the pawl (31a), when the pressing button (31b) is pressed, the pawl (31a) is advanced forward inside of the inhalation device and disengaged from the mouthpiece cover (2). After the pawl (31 a) is disengaged from the mouthpiece cover (2), the mouthpiece cover (2) can be rotated from the first position to the second position to actuate the inhalation device.

Because of the supporting part (31c) of the stopper, there is no need for a spring to enable the pressing button (31b) to be pressed. There is an end (31d) that is integrated with the supporting part and leans the housing of the inhalation device. When the pressing button is pressed, the supporting part springs over the end of said supporting part (31d) and both of the pressing button (31 b) and the pawl (31a) are advanced forward inside of the inhalation device.

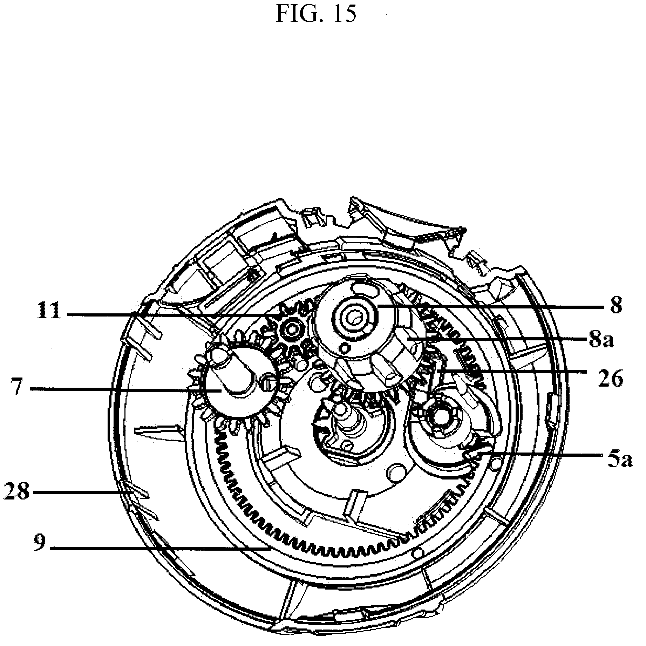

The inhalation device further comprising an another stopper (26) in the lower housing member (4b) in order to provide the opened blister in the blister package (15) which is indexed by the indexing wheel (8) to be positioned precisely. Figure 15 shows that the stopper (26) interlocks with the tooth of the indexing wheel (8) and hinders its rotation. The rotational movement of the mouthpiece cover (2) by the same angle each time the device (1) is actuated is precisely transmitted to the indexing ratchet wheel (3) by the drive gear (12) that joins with one connection point (29) of the mouthpiece cover, and therefore the indexing wheel (8) which engages with the indexing ratchet wheel (3) is rotated by the same angle each time the device (1) is actuated. The stopper component (26) positioned in the lower housing member (4b) prevents backward movement of the blister package (15) which is indexed by the indexing wheel (8) that synchronizes with the indexing ratchet wheel (3) by keeping the position of the indexing wheel (8) stable.

As can be seen in figure4, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15), which is indexed by the rotation of the indexing wheel (8), is tightly coiled on the winding wheel (13) connecting with the winding wheel gear (6) via mechanism wheel (5) and also the counter gear (9) is moved by the pinion gear (11) and the base gear (7) as a result of the rotation of the indexing wheel (8).

The lid sheet (15b) of the blister package (15) which is peeled away by the beak (16) and the base sheet (15c) are enclosed in separate compartments of the housing (10). The lid sheet (15b) that provides seal of the blister package is indexed over the beak (16) and tightly coiled on the wings (13a) of the winding wheel. The base sheet (15c) of the blister package (15) where the blister pockets (15a), each of which carries one dose of the dry powder medicament, are spaced is accumulated in the separated compartment (18a) of the housing (10). In response to each actuation of the device (1), one dose of the dry powder medicament which is prepared for inhalation after one blister pocket (15a) is opened and air entering the device through the air inlet (22) upon the inhalation of the patient provides to deliver one dose of the dry powder medicament to the patient by entraining it from the blister pocket (15a) to the mouthpiece (14).

The rotation of the indexing wheel (8) is transmitted by the pinion gear (11) to the base gear (7) engaging with the pinion gear (11) as illustrated in figure 4. A small gear which is under a base gear (7) and attached to it engages with the counter gear (9). Thus, the movement of the indexing wheel (8) is transmitted to the counter gear (9) shown in figure 4 by the pinion gear (11) and the base gear. There are numerals incrementing from 1 to 60 in the counter gear (9) displayed in figure 4. In response to each actuation of the device, the counter gear rotates and the number of the unused blister pockets remained in the device are seen through the display aperture (4c) on the lower housing member (4b).

In use, of the device described in figures 1-15, the mouthpiece (14) is exposed when the mouthpiece cover (2) is slid from the first position to the second position on the rotational path restricted on both ends by the protrusion parts (23a, 23b, 24a, 24b) on the upper housing member (4a) and the lower housing member (4b); the gear mechanism is triggered by the rotational movement of the mouthpiece cover (2) via the drive gear (12) and one dose of dry powder medicament is prepared for inhalation; the counter gear (9) is indexed and the numeral seen through the display aperture (4c) on the lower housing member (4b) is incremented. After inhalation, the mouthpiece cover (2) is moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhalation device utilized in the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, anti-inflammatories, bronchodilators, leukotirene inhibitors, PDE IV inhibitors, antitussives, diuretics, anticholinergics, hormones, xanthines and pharmaceutically acceptable combinations thereof.

The active agent comprised in the medicament in dry powder form delivered via the inhalation device pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in administration of the medicament in dry powder form which is utilized in the treatment of many respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention.

## Claims

1. An inhalation device suitable for delivery of the medicament in dry powder form from a blister package composed of a plurality of blister pockets which are spaced at equal intervals, each of which comprises medicament in dry powder form; the inhalation device comprising:
- a mouthpiece (14) enabling the patient to inhale the medicament in dry powder form from the opened blister in use;
- a gear mechanism enabling in use the blister package to be indexed and the medicament in dry powder form to become ready for inhalation;
- a rotatable mouthpiece cover (2) hiding the mouthpiece (14) and triggering the gear mechanism;
- a drive gear (12) which is a component of the gear mechanism and is joined with the mouthpiece cover (2) via a connection point of a mouthpiece cover (29), and
- a housing (10) situated between an upper housing member (4a) and a lower housing member (4b) in which the blister package and the gear mechanism are enclosed, and
- a stopper (31) for preventing the movement of the mouthpiece cover (2) **characterized in that**:
the stopper (31) consists of a pawl (31a), a pressing button (31b) and a supporting part (31c); wherein the pawl (31a) is engaged with a recess part (2c) on the inside surface of the mouthpiece cover to prevent the movement of the mouthpiece cover (2) when the mouthpiece cover (2) is in a position in which the mouthpiece (14) is completely covered, and the pressing button (31b) that moves synchronously with the pawl (31a) is pressed to enable the mouthpiece cover (2) to be rotated for the actuation of the inhalation device as the supporting part (31c) is sprung to allow the pressing button (31b) to be pressed.

2. An inhalation device according to claim 1, wherein the mouthpiece cover (2) that triggers the gear mechanism can be found in one of two positions;
- In the first position the mouthpiece cover (2) resides on the protruding parts (23a, 24a) in one end of the rotational path, the mouthpiece (14) is completely covered and the device is in standby mode,
- In the second position the mouthpiece cover resides on the protruding parts (23b, 24b) in the other end of the rotational path and one dose of the dry powder medicament is ready for inhalation due to actuation of the device

3. An inhalation device according to claim 1, wherein the mouthpiece cover (2) of the inhalation device is joined with the gear mechanism by the drive gear (12) via one connection point (29) of the mouthpiece cover.

4. An inhalation device according to claim 1 and 3, wherein the shape of the end of the drive gear (12a) that is connected to the mouthpiece cover (2) is different from the shape of the other end of the drive gear (12b).

5. An inhalation device according to claim 4, wherein the surface area of the end of drive gear (12a) that is connected to the mouthpiece cover (2) is in the range of 30-100 mm².

6. An inhalation device according to claim 5, wherein the surface area of the end of the drive gear (12a) that is connected to the mouthpiece cover (2) is in the range of 32-90 mm².

7. An inhalation device according to claim 6, wherein the surface area of the end of drive gear (12a) that is connected to the mouthpiece cover (2) is in the range of 40-80 mm².

8. An inhalation device according to claim 4, wherein the shape of the end of drive gear (12a) that is connected to the mouthpiece cover (2) is quadrangular.

9. An inhalation device according to claim 8, wherein the shape of the end of drive gear (12a) that is connected to the mouthpiece cover (2) is trapezoid.

10. The inhalation device (1) according to claim 1, wherein a restricted path is provided by protrusions (23a, 23b; 24a, 24b) on the upper (4a) and lower (4b) housing members.

11. The inhalation device (1) according to claim 1, wherein the mouthpiece cover (2) is rotationally moved by being slid on the upper (4a) and the lower (4b) housing member

12. The inhalation device (1) according to any one of the preceding claims, wherein said mouthpiece cover (2) rotates along a constant-distance path that recesses (23a, 23b; 24a, 24b) of the upper and the lower housing members define by a fixed angle in the range of 30° to 160°, according to the shape of the device, in response to the each actuation of the device.

13. The inhalation device (1) according to claim 1, wherein there is a carved part (2a) in one end of the mouthpiece cover (2) so as to allow the mouthpiece cover to rotate easily.

14. The inhalation device (1) according to claim 1, wherein all components of the gear mechanism directly or indirectly engage with each other.

15. The inhalation device (1) according to either one of claim 1 or 14, wherein said gear mechanism is composed of;
- a drive gear (12) which actuate the device (1) by transmitting the constant-angle movement of the mouthpiece cover (2) to an indexing ratchet wheel (3);
- an indexing wheel (8) which synchronizes with the indexing ratchet wheel (3) and enables the blister package (15) to be indexed in use;
- a winding wheel gear (6) which moves a winding wheel (13) via a mechanism wheel (5) upon rotation of the indexing wheel (8);
- a pinion gear (11) and a base gear (7) that transmit the movement of the indexing wheel (8) to the counter wheel (9);
- a counter gear (9) which displays the number of the unused blister pockets (15a) remained in the device (1) in use.

## Patentansprüche

1. Ein Inhalationsgerät , das für die Abgabe des Medikaments in Form eines trockenen Pulvers aus einer Blisterverpackung geeignet ist, die aus einer Vielzahl von Blistertaschen besteht, die in gleichen Abständen beabstandet sind, von denen jede ein Medikament in Form eines trockenen Pulvers umfasst; das Inhalationsgerät umfasst die Folgenden:
- ein Mundstück (14), das es den Patienten ermöglicht, das Medikament in trockener Pulverform aus dem geöffneten Blister im Einsatz zu inhalieren;
- ein Getriebe, das es bei Gebrauch der Blisterverpackung es ermöglicht, indiziert zu werden und, das den Medikamenten in trockener Pulverform es ermöglicht, zur Inhalation bereit zu werden;
- eine drehbare Mundstückabdeckung (2), die das Mundstück (14) abdeckt und das Getriebe auslöst;
- ein Antriebsrad (12), das ein Bestandteil des Getriebes ist und mit dem Mundstückabdeckung (2) über einen Verbindungspunkt einer Mundstückabdeckung (29) verbunden ist, und
- ein Gehäuse (10), das zwischen einem oberen Gehäuseelement (4a) und einem unteren Gehäuseelement (4b) liegt, in dem die Blisterverpackung und das Getriebe eingeschlossen sind, und
- ein Stopfen (31) zum Verhindern der Bewegung der Mundstückabdeckung (2), **dadurch gekennzeichnet**:
der Stopfen (31) besteht aus einer Sperrklinke (31a), einer Drucktaste (31b) und einem Stützteil (31c); die Sperrklinke (31a) ist in Eingriff mit einem Aussparungsteil (2c) an der Innenfläche der Mundstückabdeckung, um die Bewegung der Mundstückabdeckung (2) zu verhindern, wenn die Mundstückabdeckung (2) in einer Position liegt, in der das Mundstück (14) vollständig abgedeckt ist und die Drucktaste (31b) gedrückt wird, die sich synchron mit der Sperrklinke (31a) bewegt, damit die Mundstückabdeckung (2) für die Betätigung des Inhalationsgerätes gedreht werden kann, da der Stützteil (31c) gefedert wird, so dass die Drucktaste (31b) gedrückt werden kann.

2. Ein Inhalationsgerät nach Anspruch 1, wobei die Mundstückabdeckung (2), die das Getriebe auslöst, in einer von zwei Positionen zu finden ist;
- Bei der ersten Position befindet sich die Mundstückabdeckung (2) auf den vorstehenden Teilen (23 a, 24a) an einem Ende des Drehpfades und das Mundstück (14) ist vollständig abgedeckt und das Gerät ist im Standby-Modus;
- Bei der zweiten Position befindet sich die Mundstückabdeckung auf den vorstehenden Teilen (23b, 24b) am anderen Ende des Drehpfades und eine Dosis des Trockenpulvermedikaments ist durch Betätigung des Geräts zur Inhalation bereit.

3. Ein Inhalationsgerät nach Anspruch 1, wobei die Mundstückabdeckung (2) des Inhalationsgerätes über einen Verbindungspunkt (29) der Mundstückabdeckung mit dem Getriebe durch das Antriebsrad (12) verbunden ist.

4. Ein Inhalationsgerät nach Anspruch 1 und 3, wobei die Form des Endes des Antriebsrades (12a), das mit der Mundstückabdeckung (2) verbunden ist, von der Form des anderen Endes des Antriebsrades (12b) verschieden ist.

5. Ein Inhalationsgerät nach Anspruch 4, wobei der Oberflächenbereich des Endes des Antriebsrades (12a), das mit der Mundstückabdeckung (2) verbunden ist, im Bereich von 30-100 mm2 liegt.

6. Ein Inhalationsgerät nach Anspruch 5, wobei der Oberflächenbereich des Endes des Antriebsrades (12a), das mit der Mundstückabdeckung (2) verbunden ist, im Bereich von 32-90 mm2 liegt.

7. Ein Inhalationsgerät nach Anspruch 6, wobei der Oberflächenbereich des Endes des Antriebsrades (12a), das mit der Mundstückabdeckung (2) verbunden ist, im Bereich von 40-80 mm2 liegt.

8. Ein Inhalationsgerät nach Anspruch 4, wobei die Form des Endes des Antriebsrades (12a), das mit der Mundstückabdeckung (2) verbunden ist, viereckig ist.

9. Ein Inhalationsgerät nach Anspruch 8, wobei die Form des Endes des Antriebsrades (12a), das mit der Mundstückabdeckung (2) verbunden ist, trapezförmig ist.

10. Das Inhalationsgerät (1) nach Anspruch 1, wobei ein eingeschränkter Pfad durch Vorsprüngen (23a, 23b; 24a, 24b) auf den oberen (4a) und unteren (4b) Gehäuseelementen vorgesehen wird.

11. Das Inhalationsgerät (1) nach Anspruch 1, wobei sich die Mundstückabdeckung (2) dreht, indem sie auf das obere (4a) und das untere (4b) Gehäuseelement geschoben wird.

12. Das Inhalationsgerät (1) nach einem der vorhergehenden Ansprüche, wobei die genannte Mundstückabdeckung (2) sich entlang eines konstanten Abstandes dreht, so dass Aussparungen (23a, 23b; 24a, 24b) der oberen und unteren Gehäuseelemente um einen festen Winkel im Bereich von 30° bis 160° je nach der Form des Gerätes in Reaktion auf die jede Betätigung des Geräts definiert werden.

13. Das Inhalationsgerät (1) nach Anspruch 1, wobei es ein geschnitzter Teil (2a) an einem Ende der Mundstückabdeckung (2) gibt, damit die Mundstückabdeckung leicht drehen kann.

14. Das Inhalationsgerät (1) nach Anspruch 1, wobei alle Bestandteile des Getriebes direkt oder indirekt miteinander in Eingriff stehen.

15. Das Inhalationsgerät (1) nach einem der Ansprüche 1 oder 14, wobei das erwähnte Getriebe aus Folgenden besteht;
- ein Antriebsrad (12), das das Gerät (1) durch Übertragen der konstanten Winkelbewegung der Mundstückabdeckung (2) an ein Indizierungssperrrad (3) betätigt;
- ein Indizierungsrad (8), das mit dem Indizierungssperrrad (3) synchronisiert wird und der Blisterverpackung (15) es ermöglicht, im Gebrauch indiziert zu werden;
- ein Wickelzahnrad (6), das das Wickelrad (13) über den Radmechanismus (5) beim Drehen des Indizierungsrades (8) bewegt;
- ein Ritzel (11) und Basiszahnrad (7), die die Bewegung des Indizierungsrades (8) zu einem Gegenzahnrad (9) überträgt;
- ein Gegenzahnrad (9), das die Anzahl der nicht verwendeten und im Gerät (1) verbleibender Blistertaschen (15a) zeigt.

## Revendications

1. Un inhalateur approprié pour délivrer le médicament sous la forme de poudre sèche à partir d'un emballage coque composée d'une pluralité de poches blisters qui sont espacées à des intervalles égaux, dont chacune comprend le médicament sous la forme de poudre sèche et ; ledit inhalateur comprenant :
- un embout (14) permettant au patient d'inhaler le médicament sous la forme de poudre sèche depuis le blister ouvert en cours d'utilisation ;
- un mécanisme d'engrenage permettant l'emballage blister à être indexé en cours d'utilisation et le médicament sous la forme de poudre sèche à être prêt pour l'inhalation ;
- une couverture rotative de l'embout (2) cachant l'embout (14) et déclenchant le mécanisme d'engrenage ;
- un engrenage d'entrainement (12) qui est un composant du mécanisme d'engrenage et qui est relié à la couverture de l'embout (2) via un point de connexion de la couverture de l'embout (29), et
- un boîtier (10) situé entre l'élément de boîtier supérieur (4a) et l'élément de boîtier inférieur (4b), dans lequel l'emballage coque et le mécanisme d'engrenage sont enfermés, et
- une butée (31) pour empêcher le mouvement de la couverture de l'embout (2), **caractérisé en ce que :**
la butée (31) est composé d'un cliquet (31a), un bouton à presser (31b) et une partie de soutien (31c) ;
dans lequel le cliquet (31a) est engagé à la partie de cavité (2c) sur la surface intérieure de la couverture de l'embout pour empêcher le mouvement de la couverture de l'embout (2) lorsque la couverture de l'embout (2) est dans une telle position que l'embout (14) est couvert complètement,
et le bouton à presser (31b) mouvant synchroniquement avec le cliquet (31a) est pressé pour permettre la couverture de l'embout (2) à être tournée pour l'actionnement de l'inhalateur au moment où la partie de soutien (31c) est jaillie pour permettre le bouton à presser (31 a) à être pressé.

2. Un inhalateur selon la revendication 1, dans lequel la couverture de l'embout (2) déclenchant le mécanisme d'engrenage peut se trouver l'un des deux positions ;
- la première position, la couverture de l'embout (2) se trouve sur les parties en saillie (23a, 24a) sur une extrémité de la route de rotation lorsque la couverture de l'embout (14) est complètement couvert et le dispositif est en mode veille;
- la seconde position, la couverture de l'embout se trouve sur les parties en saillie (23b, 24b) de l'autre extrémité de la route de rotation et une dose du médicament en poudre sèche devient prête pour l'inhalation, à cause de l'actionnement du dispositif.

3. Un inhalateur selon la revendication 1, dans lequel la couverture de l'embout (2) de l'inhalateur est reliée au mécanisme d'engrenage par l'engrenage d'entrainement (12) via un point de connexion de la couverture de l'embout (29).

4. Un inhalateur selon les revendications 1 et 3, dans lequel la forme du bout de l'engrenage d'entrainement (12a) qui est relié à la couverture de l'embout (2) est différente de l'autre bout de l'engrenage d'entrainement (12b).

5. Un inhalateur selon la revendication 4, dans lequel la superficie du bout de l'engrenage d'entrainement (12a) qui est relié à la couverture de l'embout (2) est dans la plage de 30-100 mm².

6. Un inhalateur selon la revendication 5, dans lequel la superficie du bout de l'engrenage d'entrainement (12a) qui est relié à la couverture de l'embout (2) est dans la plage de 32-90 mm².

7. Un inhalateur selon la revendication 6, dans lequel la superficie du bout de l'engrenage d'entrainement (12a) qui est relié à la couverture de l'embout (2) est dans la plage de 40-80 mm².

8. Un inhalateur selon la revendication 4, dans lequel la forme du bout de l'engrenage d'entrainement (12a) qui est relié à la couverture de l'embout (2) est quadrangulaire.

9. Un inhalateur selon la revendication 8, dans lequel la forme du bout de l'engrenage d'entrainement (12a) qui est relié à la couverture de l'embout (2) est trapèze.

10. L'inhalateur (1) selon la revendication 1, dans lequel une route restreinte est prévue par les saillies (23a, 23b ; 24a, 24b) sur les éléments de boîtier supérieur (4a) et inférieur (4b).

11. L'inhalateur (1) selon la revendication 1, dans lequel la couverture de l'embout (2) est déplacée par rotation par glissement sur l'élément de boîtier supérieur (4a) et inférieur (4b).

12. L'inhalateur (1) selon l'une quelconque des revendications précédentes, dans lequel ladite couverture de l'embout (2) tourne le long d'une route à distance constante que les cavités (23a, 23b; 24a, 24b) de l'élément de boîtier supérieur et inférieur définie par un angle fixe dans la plage de 30 ° à 160 °, suivant la forme du dispositif, en réponse à chaque actionnement du dispositif.

13. L'inhalateur (1) selon la revendication 1, dans lequel il y a une partie gravée (2a) à une extrémité de la couverture de l'embout (2) afin de permettre la couverture de l'embout à tourner facilement.

14. L'inhalateur (1) selon la revendication 1, dans lequel tous les composants du mécanisme d'engrenage s'engagent les uns avec les autres directement ou indirectement.

15. L'inhalateur (1) selon l'une quelconque des revendications 1 ou 14, dans lequel ledit mécanisme d'engrenage est composé de :
- un engrenage d'entrainement (12) qui enclenche l'appareil (1) en transmettant le mouvement d'un angle constant de la couverture de l'embout (2) à une roue à rochet d'indexation (3) ;
- une roue d'indexation (8) qui synchronise avec la roue à rochet d'indexation (3) et permet l'emballage coque (15) à être indexé en cours d'utilisation ;
- un engrenage de roue d'enroulement (6) qui déplace une roue d'enroulement (13) par un mécanisme de roue (5) sur la rotation de la roue d'indexation (8) ;
- un engrenage à pignons (11) et un engrenage de base (7) qui transmettent le mouvement du la roue d'indexation (8) à la roue de contrepoussée (9) ;
- un roue de contrepoussée (9) qui affiche le nombre des poches blisters (15a) inutilisées restantes dans l'appareil (1) en cours d'utilisation.
